(19) Europäisches Patentamt
European Patent Office
Office européen des brevets

(11) **EP 4 442 809 A1**

(12) **EUROPEAN PATENT APPLICATION**
published in accordance with Art. 153(4) EPC

(43) Date of publication:
**09.10.2024 Bulletin 2024/41**

(21) Application number: **22901183.8**

(22) Date of filing: **25.11.2022**

(51) International Patent Classification (IPC):
**C12N 5/07** (2010.01)    **G01N 1/28** (2006.01)
**C12M 1/00** (2006.01)

(52) Cooperative Patent Classification (CPC):
**C12M 1/00; C12N 5/06; G01N 1/28**

(86) International application number:
**PCT/JP2022/043479**

(87) International publication number:
**WO 2023/100750 (08.06.2023 Gazette 2023/23)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **30.11.2021 JP 2021193995**

(71) Applicant: **RIKEN**
**Wako-shi, Saitama 351-0198 (JP)**

(72) Inventors:
• **SHIROGUCHI, Katsuyuki**
**Wako-shi, Saitama 351-0198 (JP)**
• **HOJO, Nozomi**
**Wako-shi, Saitama 351-0198 (JP)**

(74) Representative: **Graf von Stosch**
**Patentanwaltsgesellschaft mbH**
**Prinzregentenstraße 22**
**80538 München (DE)**

(54) **SAMPLE PREPARATION SYSTEM, SAMPLE PREPARATION METHOD, AND SAMPLE ANALYSIS SYSTEM**

(57) A sample preparation system (100) includes a position identification section (34) that associates a plurality of cells constituting a cell group with the respective plurality of cells constituting the cell group in which at least a part of junctions between adjacent ones of the plurality of cells is disconnected, in a dispersion process of (i) disconnecting at least the part of the junctions between the adjacent ones of the plurality of cells in the cell group in which the plurality of cells are joined to each other and (ii) dispersing the cell group. This allows the realization of a technique of collecting a cell and specifying the position of the collected cell in an original cell group.

FIG. 1

EP 4 442 809 A1

**Description**

Technical Field

The present invention relates to a sample preparation system, a sample preparation method, and a sample analysis system.

Background Art

In a case where the properties of individual cells included in a cell group, such as a cultured cell mass or a tissue, are analyzed, a single target cell is picked from the cell group and subjected to an analysis. As a technique for collecting a single target cell from a cell group, a cell transport system disclosed in Patent Literature 1 is known.
In the system disclosed in Patent Literature 1, a single cell is collected from a cell group and then discharged into a PCR tube, while the inside of a Petri dish is observed by a microscope.

Citation List

[Patent Literature]

[Patent Literature 1]

Japanese Patent Application Publication Tokukai No. 2018-68169

Summary of Invention

Technical Problem

In a case where a cell that functions systematically with an adjacent cell, such as a cancer cell, is analyzed or in a case where an interaction between adjacent cells is analyzed, not only the properties of individual cells but also positional information on the positions of these cells in a cell group is important. Therefore, in a cell analysis, it is preferable that positional information on cells be obtained together with the properties of the individual cells.
In the system disclosed in Patent Literature 1, a cell is collected under microscopic observation. In this case, each cell is dispersed or separated so that each cell can be easily collected. Therefore, original positional information on each cell in a cell group is lost, and it is therefore difficult to obtain positional information on the collected cell. In particular, in the case of a three-dimensional sample such as a cell mass or a part of a tissue, it is difficult to collect a cell while three-dimensional positional information in the sample is held.
The present invention has been made in order to solve the above problem, and the object thereof is to realize a technique for preparing a sample containing a cell to be subjected to a cell analysis, while tracking the positions of individual cells constituting a cell group.

Solution to Problem

A sample preparation system in accordance with an aspect of the present invention includes: a tracking section that associates a plurality of cells constituting a cell group with the respective plurality of cells constituting the cell group in which at least a part of junctions between adjacent ones of the plurality of cells is disconnected, in a dispersion process of (i) disconnecting at least the part of the junctions between the adjacent ones of the plurality of cells in the cell group in which the plurality of cells are joined to each other and (ii) dispersing the cell group.
A sample analysis system in accordance with an aspect of the present invention includes: a sample preparation system in accordance with an aspect of the present invention and an analysis device that analyzes a sample prepared by the sample preparation system.
A sample preparation method in accordance with an aspect of the present invention includes: a step of (i) disconnecting at least a part of junctions between adjacent ones of a plurality of cells in a cell group in which the plurality of cells are joined to each other and (ii) dispersing the cell group, the step being a step of dispersing the cell group while associating the plurality of cells constituting the cell group with the respective plurality of cells constituting the cell group in which at least the part of the junctions between the adjacent ones of the plurality of cells is disconnected.

Advantageous Effects of Invention

An aspect of the present invention makes it possible to prepare a sample containing a cell to be subjected to a cell analysis, while tracking the positions of individual cells constituting a cell group.

Brief Description of Drawings

Fig. 1 is a cross-sectional view taken along a line and schematically illustrating a sample preparation system in accordance with an embodiment of the present invention.
Fig. 2 is a block diagram illustrating a configuration of a main part of the sample preparation system illustrated in Fig. 1.
Fig. 3 is a view illustrating a flow of a sample collection process in which the sample preparation system illustrated in Fig. 1 is used.
Fig. 4 is a three-dimensional fluorescence image of a cell mass immediately after staining, in Example 1.
Fig. 5 is a view illustrating changes in position of cell nuclei with elapse of an enzymatic cell dispersion treatment time, in Example 1.
Fig. 6 is a view illustrating changes in position of the cell nuclei in the process of dispersing cells with use of a convex lens, in Example 1.
Fig. 7 is a view illustrating changes in position of the cell nuclei with movement of the convex lens, in Example 1.
Fig. 8 is a view illustrating a manner in which a single cell was separated from the cell mass with use of a glass needle, in Example 1.
Fig. 9 is a view illustrating the three-dimensional position and the track of movement of a collected cell in dispersion, separation, and collection processes, in Example 1.
Fig. 10 is a three-dimensional fluorescence image of cell nuclei immediately after staining, in Example 2.
Fig. 11 is a view illustrating changes in position of the cell nuclei with elapse of a cell dispersion solution treatment time, in Example 2.
Fig. 12 is a view illustrating changes in position of the cell nuclei in the process of dispersing cells with use of a convex lens, in Example 2.
Fig. 13 is a view illustrating the positions, on a two-dimensional plane, of the cells before and after the dispersion of the cells, in Example 2.

Description of Embodiments

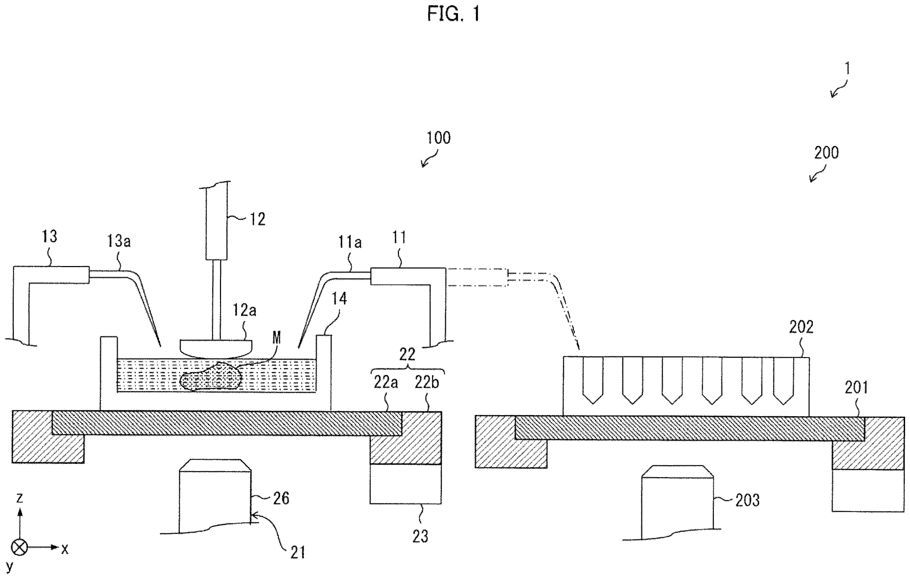

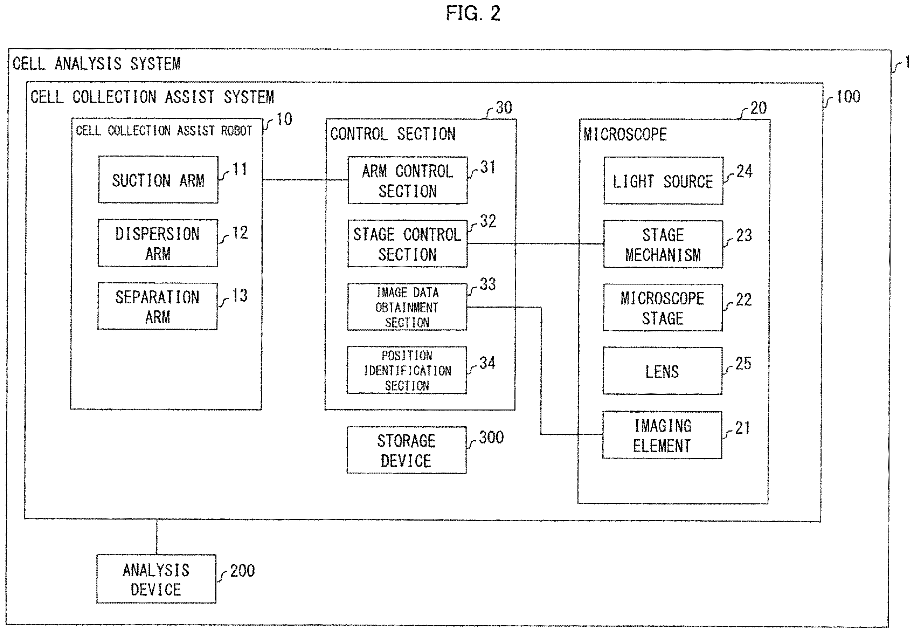

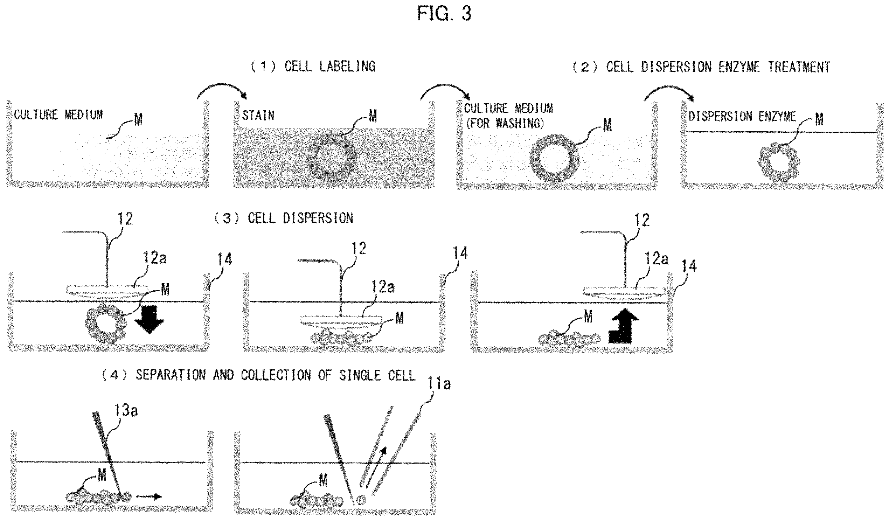

The following description will discuss a sample preparation system 100 in accordance with an embodiment of the present invention with reference to Figs. 1 to 3. Fig. 1 is a cross-sectional view taken along a line and schematically illustrating the sample preparation system 100 in accordance with an embodiment of the present invention. Fig. 2 is a block diagram illustrating a configuration of a main part of the sample preparation system 100 illustrated in Fig. 1. Fig. 3 is a view illustrating a flow of a sample collection process in which the sample preparation system 100 illustrated in Fig. 1 is used.

[Sample preparation system 100]

The sample preparation system 100 is a system that prepares a sample to be analyzed, from a cell group in which a plurality of cells are joined to each other. Examples of the "sample" to be analyzed include: a cluster constituted by a smaller number of cells than of the cells constituting the cell group; a small cell group; a single cell; a part of constituent elements constituting a cell; and a part of components contained in a cell. Further, an expression "preparing a sample" is intended to mean bringing the sample into a state in which the sample can be analyzed. As examples, the expression "preparing a sample" includes: collecting (picking) a single cell from the cell group and transporting the single cell onto a plate to be subjected to a cell analysis; and separating, in a solution in which the cell group is contained, a cell mass to be analyzed so that the cell mass can be analyzed in the solution. In the present embodiment, a case where a single cell is collected as the sample is mainly described as an example. The sample preparation system 100 constitutes a sample analysis system 1 together with an analysis device 200. In the sample analysis system 1, a cell picked by the sample preparation system 100 is transported to the analysis device 200 and subjected to a sample analysis.

The sample preparation system 100 includes a sample preparation robot 10, a microscope 20, a control section 30, and a storage device 300. The sample preparation system 100 is used to collect at least a part of a cell group M in a Petri dish (container) 14. A container in which the cell group M collected by the sample preparation system 100 is held is not limited to the Petri dish 14, and only needs to be a container in which the cell group M can be held, such as a tube or a plate. Such a container is preferably made of glass or a resin each of which has light transmitting properties with respect to observation light of the microscope 20 (in the present embodiment, each of which is transparent).

In the sample preparation system 100, the cell group in which a cell to be collected is included is a cell group in which a plurality of cells are joined to each other. Such a cell group may be a two-dimensional structure such as a sheet-like cell cluster or may be a three-dimensional structure such as a cell mass, a tissue, or an organoid.

In the sample preparation system 100, the cell group M is preferably held in the Petri dish 14 together with a solution containing bead-like solid particles. The solid particles contained in the solution are, for example, resin beads. A culture medium may also be contained in the solution.

(Sample preparation robot 10)

The sample preparation robot 10 includes three robot arms, that is, a suction arm (collection arm) 11, a dispersion arm 12, and a separation arm 13.

<Suction arm 11>

The suction arm 11 is used to suction a target cell from the cell group M in the Petri dish 14 and discharge the target cell onto a plate 202 of the analysis device 200. Note that, instead of the suction arm 11, another collection arm that can collect the target cell from the cell group M may be used. Examples of the another arm include a needle that is inserted into a cell so as to collect the cell. In the present embodiment, a case where the suction arm 11 is used as a collection arm is described as an example.

The suction arm 11 suctions at least a part of target cells from the cell group M dispersed on a two-dimensional plane (xy plane) in the Petri dish 14, that is, a bottom surface of the Petri dish 14. For example, the suction arm 11 suctions a cell in the cell group M dispersed in the Petri dish 14, the cell junction of which cell is separated and which cell is disconnected.

A needle 11a is attached to an extreme end of the suction arm 11. The suction arm 11 is configured to be rotatable in an x axis direction in Fig. 1. This makes it possible for the needle 11a of the suction arm 11 to access both of the Petri dish 14 of the sample preparation system 100 and the plate 202 of the analysis device 200 which are adjacently placed.

The suction arm 11 has a plurality of joints, and is configured such that the position of an extreme end of the needle 11a can be adjusted in each of the x axis direction, a y axis direction, and a z axis direction by control of movement of the joints. In particular, by moving up and down the position of the extreme end of the needle in the z axis direction, it is possible to insert or extract the extreme end of the needle 11a into or from the Petri dish 14 without moving a microscope stage 22, and possible to insert or extract the extreme end of the needle 11a into or from the plate 202.

The suction arm 11 is configured such that the position of the needle 11a can be adjusted either manually or in a motorized manner. Note that the suction arm 11 includes a plurality of motors (not illustrated) so that the suction arm 11 is driven in a motorized manner. In a case where the suction arm 11 is driven in a motorized manner, the suction arm 11 is controlled by an arm control section 31 included in the control section 30 of the sample preparation system 100. Note that an aspect of the plurality of motors is not limited, and the plurality of motors may be, for example, stepping motors or servo motors.

The needle 11a is a tubular member whose both ends are opened. One of the ends of the needle 11a is connected, in an air-tight state, to piping connected to a pump (not illustrated). Note that, although a detailed description of the pump is omitted, the pump is configured to be capable of suctioning and discharging a minute volume of fluid (gas or liquid). Since the pump and the needle 11a are connected in an air-tight state, the needle 11a can suction or discharge a cell in response to suction or discharge of the fluid each of which is carried out by the pump.

The needle 11a can have a tapered shape in which the needle 11a is tapered toward the extreme end. The opening diameter and inner diameter of the needle 11a each only need to be a diameter which allows a cell to be suctioned or discharged, and can be each set, as appropriate, in accordance with the size of a target cell. The needle 11a only needs to be made of glass or a resin each of which has light transmitting properties with respect to the observation light from the microscope 20 (in the present embodiment, each of which is transparent).

<Dispersion arm 12>

The dispersion arm 12 is used in such a manner that an extreme end thereof is pressed against the cell group M in the Petri dish 14 so that at least a part of junctions between adjacent cells in the cell group M is disconnected and the cell group M is dispersed. This dispersion process is intended, for example, to disperse the cell group into smaller clusters. Note, here, that an expression "dispersing the cell group into smaller clusters" is intended to mean dividing the cell group into small cell populations (clusters) each of which is constituted by a smaller number of cells than of the cells constituting the cell group. As other examples, the expression "dispersing the cell group into smaller clusters" includes: dividing a single cell group into two or more clusters; and causing at least a part of the cells included in the cell group to be away from adjacent cells.

As described below, in the dispersion process described above, the cells constituting the cell group are associated with the respective cells constituting the clusters. Thus, the expression "dispersing the cell group into smaller clusters" can mean dispersing each cell or cell masses to such a degree that such association can be carried out. Moreover, in a case where the cell group is a three-dimensional structure, the dispersion process described above can also include spreading the three-dimensional structure onto the two-dimensional plane. Further, the dispersion process described above can include stacking up and down the individual cells constituting the cell group such that the cells are three-dimensionally dispersed.

The cell group M against which the extreme end of the dispersion arm 12 is pressed may be previously treated with a dispersion enzyme in the Petri dish 14 so as to be in a state in which the cell group M is easily dispersed. Note that a method for dispersing the cell group M is not limited to a configuration in which the dispersion arm 12 is used. As described below, another conventionally known cell dispersion method may be used. In a case where another cell dispersion method is used, the sample preparation system 100 may not include the dispersion arm 12.

The dispersion arm 12 has, at the extreme end thereof, a surface that presses the cell group M from the top of the cell group M in the direction of the bottom surface of the Petri dish 14. In the present embodiment, a configuration in which a convex lens 12a is attached to the extreme end of the dispersion arm 12 is described as an example. By moving the convex lens 12a in the z axis direction, the dispersion arm 12 presses the convex lens 12a against the cell group M from the top of the cell group M in the direction of the bottom surface of the Petri dish 14, and thereby disperses the cell group M. In a case where the cell group M is a three-dimensional structure such as a cell mass, an organoid, or a part of a tissue, it is possible to disperse the cell group M on the two-dimensional plane by pressing the convex lens 12a against the cell group M.

The convex lens 12a has a curved surface in a protruding shape (protruding part). Thus, even in a case where the posture of the convex lens 12a is not parallel to the Petri dish 14, an end of the convex lens 12a does not interfere with the Petri dish 14, and it is therefore possible to appropriately press the convex lens 12a against the cell group M. Moreover, it is possible to easily carry out positioning of the convex lens 12a with respect to the cell group M, on the basis of the position of the protruding part. The convex lens 12a is preferably pressed against the cell group M so that an extreme end of the protruding part (the lowest point of the convex lens 12a) is first brought into contact with the cell group M.

By moving the dispersion arm 12 so that the convex lens 12a is moved in the x axis direction or the y axis direction, positioning of the convex lens 12a may be carried out so that the convex lens 12a can be appropriately pressed against the cell group M. Note also that, in a case where the size of the convex lens 12a in the x axis direction or the y axis direction is smaller than that of the cell group M, the convex lens 12a may be moved in the x axis direction or the y axis direction on the cell group M so that the convex lens 12a can presses the entire cell group M.

In the Petri dish 14, a solution containing bead-like solid particles is held together with the cell group M. Therefore, in a case where the convex lens 12a is moved in the z axis direction in the solution, a flow is generated in the solution, so that the solid particles move. Therefore, by observing movement of the solid particles, it is possible to carry out positioning of the convex lens 12a with respect to the cell group M. For example, in a case where the convex lens 12a is moved in the z axis direction in the solution, the solid particles move in a centripetal or centrifugal direction around the lowest point of the convex lens 12a on an xy plane near the bottom surface of the Petri dish 14. Then, by observing this movement of the solid particles (flow of the solution), it is possible to identify the lowest point of the convex lens 12a. Alternatively, positioning of the convex lens 12a may be carried out with reference to, as an index, a Newton's rings-like image observed when the convex lens 12a is brought close to the bottom surface of the container.

The dispersion arm 12 is configured such that the position of the convex lens 12a can be adjusted either manually or in a motorized manner. Note that the dispersion arm 12 includes a plurality of motors (not illustrated) so that the dispersion arm 12 is driven in a motorized manner. In a case where the dispersion arm 12 is driven in a motorized manner, the dispersion arm 12 is controlled by the arm control section 31 included in the control section 30 of the sample preparation system 100. Note that an aspect of the plurality of motors is not limited, and the plurality of motors may be, for example, stepping motors or servo motors.

The convex lens 12a only needs to have the protruding part that protrudes in the direction in which the convex lens 12a presses the cell group M. The convex lens 12a may not function as a lens. Furthermore, the shape, size, and material of the convex lens 12a are not particularly limited, provided that the convex lens 12a has the curved surface in a protruding shape. Instead of the convex lens 12a, a glass substrate or a resin substrate can also be used. The convex lens 12a only needs to be made of glass or a resin each of which has light transmitting properties with respect to the observation light from the microscope 20 (in the present embodiment, each of which is transparent). The size of the convex lens 12a in the x axis direction and the y axis direction is preferably a size which allows the entre cell group M to be covered (the size of the convex lens 12a in the x axis direction and the y axis direction is preferably greater than that of the cell group M). However, since the convex lens 12a can also be moved in the x axis direction and the y axis direction and pressed against the cell group M, the size of the convex lens 12a in the x axis direction and the y axis direction may be smaller than that of the cell group M.

The shape of the convex lens 12a is preferably a shape which does not cause the cell group M to translate in a case where the convex lens 12a is pressed against the cell group M. It is not preferable that the cell group M translate in a case where the convex lens 12a is pressed against the cell group M. This is because the cell group M falls outside the field of view of the microscope 20 and can no longer be tracked or the cell group M can no longer be dispersed appropriately.

In order that the cell group M can be appropriately dispersed, the convex lens 12a is preferably moved in the z axis direction and pressed against the cell group M in a state in which the convex lens 12a is parallel to the bottom surface of the Petri dish 14. However, a deviation from a degree of parallelism is allowed, provided that the convex lens 12a has an appropriate shape. That is, the convex lens 12a is preferably configured such that, even in a case where the convex lens 12a is pressed against the cell group M in a state in which the convex lens 12a is not parallel, the cell group M does not fall outside the field of view of the microscope 20 and the cell group M can be appropriately dispersed. In a case where such a shape of the convex lens 12a is studied, the following is found.

The radius of the convex lens 12a is regarded as "r". In this case, the radius of curvature R of the convex lens 12a which radius of curvature R allows a deviation (0) of the degree of parallelism of the convex lens 12a from the bottom surface of the Petri dish 14 preferably satisfies the following expression.

$$R \leq r/\sin\theta \cdots (\text{expression } 1)$$

Note that the upper limit of the radius r of the convex lens 12a depends on a space around the convex lens 12a (such as the size of the Petri dish 14).

The radius r of the convex lens 12a is preferably greater than the longer axis radius $L_1$ of the cell group M. Therefore, the radius r preferably satisfies the following expression.

$$L_1 \leq r \leq R \cdots (\text{expression } 2)$$

Then, in order that the cell group M is prevented from translating in a case where the convex lens 12a is pressed against the cell group M, the radius r needs to be greater than a constant value $L_2$ ($\geq L_1$) (the value of the $L_2$ varies depending on an effect of deformation of the cell group M, friction between the cell group M and the convex lens 12a or the bottom surface of the Petri dish 14, or the like). Therefore, the radius r preferably satisfies the following expression.

$$L_1 \leq L_2 \leq r \leq R \cdots (\text{expression } 3)$$

The shape of the convex lens 12a which shape allows the convex lens 12a to be appropriately pressed also against an end of the cell group M is studied. The longer axis radius of the cell group M in a case where the convex lens 12a is pressed against the cell group M is regarded as $L_3$. In this case, the distance d between (i) the lowest point of the convex lens 12a having a radius of curvature R and (ii) a lens surface at a position shifted by the longer axis radius $L_3$ from the lowest point preferably satisfies the following expression.

$$d = R\text{-}\mathrm{sqrt}(R^2\text{-}L_3^2) \cdots (\text{expression } 4)$$

Here, it is assumed that the height of a single cell is $d_0$. In this case, in order that even the end of the convex lens 12a can be pressed against the cells, d is preferably made shorter than the $d_0$. Therefore, in expression 4, for example, in a case where $L_3$ = 0.1 mm and $d_0$ = 0.01 mm, the radius of curvature R only needs to be greater than 0.5 mm.

<Separation arm 13>

The separation arm 13 is used to separate a cell junction in the cell group M dispersed in the Petri dish 14. The separation arm 13 separates the cell junction in the cell group M dispersed on the two-dimensional plane in the Petri dish 14, that is, on the bottom surface of the Petri dish 14.

A needle 13a is attached to an extreme end of the separation arm 13. The separation arm 13 is configured to be rotatable in the x axis direction in Fig. 1. The separation arm 13 has a plurality of joints, and is configured such that the position of an extreme end of the needle 13a can be adjusted in each of the x axis direction, the y axis direction, and the z axis direction by control of movement of the joints. In particular, by moving up and down the position of the extreme end of the needle in the z axis direction, it is possible to insert or extract the extreme end of the needle 13a into or from the Petri dish 14 without moving the microscope stage 22.

The separation arm 13 is configured such that the position of the needle 13a can be adjusted either manually or in a motorized manner. In a case where the separation arm 13 is manually driven, a hydraulic manipulator is used, for example. The separation arm 13 includes a plurality of motors (not illustrated) so that the separation arm 13 is driven in a motorized manner. In a case where the separation arm 13 is driven in a motorized manner, the separation arm 13 is controlled by the arm control section 31 included in the control section 30 of the sample preparation system 100. Note that an aspect of the plurality of motors is not limited, and the plurality of motors may be, for example, stepping motors or servo motors.

The needle 13a can have a tapered shape in which the needle 13a is tapered toward the extreme end. The outer diameter of the extreme end only needs to be a diameter which allows the extreme end to be inserted between cells and separate a cell junction. The outer diameter of the extreme end can be set, as appropriate, in accordance with a state of a junction in the target cell group. The needle 13a may be made of glass or a resin each of which has light transmitting properties with respect to the observation light from the microscope 20 (in the present embodiment, each of which is transparent).

(Microscope 20)

The microscope 20 includes an imaging element 21, the microscope stage 22, a stage mechanism 23, a light source 24, and a lens 25. The microscope 20 is placed on a vibration-proof table (not illustrated).

The imaging element 21 captures images of the inside of the Petri dish 14 which images are formed by the lens 25, and generates image data indicating the images. The microscope 20 is preferably a microscope having specifications suitable for a detailed observation and a detailed analysis of the cells. The image data generated by the imaging element 21 is transmitted to and stored in the storage device 300.

The imaging element 21 captures the images (microscope images) of the inside of the Petri dish 14 from when the cell group M is dispersed until a cell is suctioned. The imaging element 21 may capture the images of the inside of the Petri dish 14 before the cell group M is dispersed or at the same time as when the dispersion is started. The imaging element 21 captures the images of the inside of the Petri dish 14 in a time-lapse manner at given time intervals. The time intervals of this time-lapse capturing of the images may be set to a value which allows tracking of changes in position and state of the cell group M in the Petri dish 14. Further, the imaging element 21 may capture a moving image of the inside of the Petri dish 14. The frame rate of the moving image may be a value which allows tracking of changes in position and state of the cell group M in the Petri dish 14. The imaging element 21 generates two-dimensional image data or three-dimensional image data on the images of the inside of the Petri dish 14.

The microscope stage 22 is a stage on which the Petri dish 14 is placed. The microscope stage 22 includes a plate 22a and an outer frame 22b. The microscope stage 22 further includes the stage mechanism 23 for adjusting the position of the microscope stage 22.

The plate 22a is a plate-like member on which the Petri dish 14 is placed, and can be made of glass that has light transmitting properties with respect to the observation light from the light source 24.

The outer frame 22b is a frame-like member for supporting the plate 22a, and is shaped such that the outer frame 22b surrounds the outer edge of the plate 22a. The outer frame 22b is supported by the stage mechanism 23 schematically illustrated in Fig. 1. The position of the outer frame 22b can be adjusted with use of the stage mechanism 23.

The stage mechanism 23 is configured to be capable of translating the outer frame 22b in each of the x axis direction, the y axis direction, and the z axis direction and tilting a main surface of the outer frame 22b (main surface of the plate 22a) with respect to the xy plane.

The stage mechanism 23 is configured to be capable of adjusting the position of the outer frame 22b either manually or in a motorized manner. Note that the stage mechanism 23 includes a plurality of motors so that the stage mechanism 23 is driven in a motorized manner. In a case where the stage mechanism 23 is driven in a motorized manner, the stage mechanism 23 is controlled by a stage control section 32 included in the control section 30 of the sample preparation system 100. Note that an aspect of the plurality of motors is not limited, and the plurality of motors may be, for example, stepping motors or servo motors.

The microscope stage 22 is configured as described above. Therefore, in a state in which the microscope 20 is adjusted such that a focus is on the inside of the Petri dish 14 on the microscope stage 22, it is possible to collect a target cell from the cell group M in the Petri dish 14 without changing the relative relationship between the inside of the Petri dish 14 and the microscope 20 (that is, in a state where the focus is on the inside of the Petri dish 14). Moreover, during collection of a desired cell with use of the sample preparation robot 10, it is possible to track the desired cell in a state where the focus is on the inside of the Petri dish 14.

The light source 24 is a light source that emits the observation light (illumination light, excitation light, or the like) with respect to the Petri dish 14. In order that bright field images of the inside of the Petri dish 14 are obtained, the light source 24 is disposed above the microscope stage 22 because an objective lens 26 and the imaging element 21 are disposed below the microscope stage 22. In this case, the microscope stage 22, the Petri dish 14, the needle 11a, the convex lens 12a, and the needle 13a preferably have light transmitting properties with respect to the light from the light source 24. Note that, in contrast to the configuration described above, the light source 24 may be disposed below the microscope stage 22, and the objective lens 26 and the imaging element 21 may be disposed above the microscope stage 22.

In a case where fluorescence images of the inside of the Petri dish 14 are obtained, the following configuration can be employed, as an example. That is, the objective lens 26 and the imaging element 21 are disposed below the microscope stage 22, and excitation light from the light source 24 is emitted from below or from the side of the cell group M in the Petri dish 14. As another example, the following configuration may be employed. That is, the objective lens 26 and the imaging element 21 are disposed above the microscope stage 22, and the excitation light from the light source 24 is emitted from above or from the side of the cell group M in the Petri dish 14. Note that a configuration of an optical system including the light source 24, the objective lens 26, and the imaging element 21 is not limited to these configurations. That is, the position of the light source 24 is not limited, provided that the above-described direction of emission of the excitation light can be realized. Further, the position of the imaging element 21 is not limited, provided that light transmitted by the objective lens 26 enters the imaging element 21.

The lens 25 is composed of a plurality of lenses, and a part of the plurality of lenses constitutes the objective lens 26. The lens 25 forms an image of the inside of the Petri dish 14 on a light receiving surface of the imaging element 21.

(Control section 30)

The control section 30 includes the arm control section (adjustment mechanism) 31, the stage control section 32, an image data obtainment section 33, and a position identification section (tracking section) 34.

The arm control section 31 controls driving of three arms included in the sample preparation robot 10 so that the position of the extreme end of each arm is adjusted. In particular, the arm control section 31 may drive the dispersion arm 12 and thereby carry out positioning of the convex lens 12a of the dispersion arm 12 so that the lowest point of the convex lens 12a is located at a position at which the lowest point faces the center or the vicinity of the center of the cell group M. The arm control section 31 may also control movement of the convex lens 12a in the z axis direction on the basis of information indicating a position at which the lowest point of the convex lens 12a is in contact with the bottom surface of the Petri dish 14 (the height of the lens is 0) (reference position).

The arm control section 31 may cause the convex lens 12a to be moved in the z axis direction and pressed against the cell group M, and then cause the convex lens 12a to be moved in the x axis direction and the y axis direction. Further, the arm control section 31 may cause the convex lens 12a to be vibrated in the z axis direction or may cause the convex lens 12a to be vibrated in the x axis direction or the y axis direction. In a case where the cell group M is a three-dimensional structure, the arm control section 31 preferably carries out control so that the convex lens 12a is pressed against the cell group M until the cell group M is dispersed on the two-dimensional plane in the Petri dish 14,

The arm control section 31 causes the convex lens 12a to be pressed against the cell group M so that the cell group M is dispersed, and then causes the convex lens 12a to be moved in the direction away from the cell group M in the z axis direction. In this case, the arm control section 31 may cause the convex lens 12a to be moved in the direction away from the cell group M in the x axis direction or the y axis direction, and then cause the convex lens 12a to be moved in the direction away from the cell group M in the z axis direction. Thereafter, the arm control section 31 drives the separation arm 13 so as to separate a cell junction, and drives the suction arm 11 so as to suction a target cell. The arm control section 31 may carry out control so that (i) the convex lens 12a is pressed against the cell group M so that the cell group M is dispersed and then (ii) the convex lens 12a is further pressed against the dispersed cell group M so that the cell group M is divided into clusters each constituted by a smaller number of cells.

The arm control section 31 preferably carries out control so that, in a case where the convex lens 12a is pressed against the cell group M or the convex lens 12a is moved in the direction away from the cell group M, the cell group M does not translate with movement of the convex lens 12a and does not fall outside the field of view of the microscope 20. That is, the arm control section 31 preferably causes the convex lens 12a to be moved at such a speed that the cell group M does not translate and does not fall outside the field of view of the microscope 20.

Note that, from the viewpoint of obtaining tracking images of the cells in the cell group M with use of the imaging element 21, the speed of movement of the convex lens 12a is preferably slower than the intervals of the time-lapse capturing of the images by the imaging element 21 or the frame rate of the moving image. The arm control section 31, for example, causes the convex lens 12a to be moved in the z axis direction by several micrometers per second.

The stage control section 32 controls the stage mechanism 23 of the microscope stage 22 so that the position of each of the plate 22a and the outer frame 22b, that is, the position of the Petri dish 14 is adjusted. The stage control section 32 may control the stage mechanism 23 so that the position of the Petri dish 14 with respect to the imaging element 21 can be adjusted and the position of the Petri dish 14 with respect to three arms included in the sample preparation robot 10 is adjusted.

Note that the position of the extreme end of each of the three arms included in the sample preparation robot 10 relative to the position of the Petri dish 14 may be adjusted by one of the arm control section 31 and the stage control section 32 or both of the arm control section 31 and the stage control section 32. That is, the arm control section 31 may drive each arm so that the position of the extreme end of each arm with respect to the position of the Petri dish 14 is adjusted. Alternatively, the stage control section 32 may drive the microscope stage 22 so that the position of the Petri dish 14 with respect to the position of the extreme end of each arm is adjusted. Alternatively, both of these methods may be employed. Therefore, both of the arm control section 31 and the stage control section 32 can function as an adjustment mechanism of the sample preparation system 100.

The image data obtainment section 33 obtains the image data generated by the imaging element 21, and transmits the image data to the position identification section 34 and the storage device 300. The image data obtainment section 33 may obtain the image data each time the imaging element 21 generates the image data or may obtain the image data at a given timing.

In the dispersion process, the position identification section 34 associates the cells constituting the cell group M with the respective cells constituting the cell group in which at least a part of junctions between adjacent cells is disconnected. For example, the position identification section 34 identifies the position of each cell in the cell group M from before the cell group M is dispersed until a cell is collected, with reference to the image data obtained by the image data obtainment section 33. The position identification section 34 can identify the original position, in the cell group M, of the cell collected by the suction arm 11. The position identification section 34 (i) generates, from the obtained image data, a time-series image from when the cell group M is dispersed until at least a part of the cell group is suctioned, (ii) associates each cell between the images, and (iii) tracks the position of each cell at each time point. For example, the position identification section 34 tracks the position of each cell at each time point by reconstructing, as a three-dimensional image, a result of scanning each pixel (x, y, z) obtained from a confocal microscope. In this manner, the original position, in the cell group M, of each cell may be identified. The position identification section 34 transmits, to the storage device 300, positional data indicating the identified position of each cell.

Note that, instead of an aspect in which the images captured during the dispersion process are referred to as described above, the position identification section 34 may associate the position of each cell before and after the dispersion process with use of a device that obtains a signal for detecting the position of each cell. For example, the position identification section 34 calculates the position of each cell at each time point from information on each pixel obtained from the confocal microscope, not images the information on each pixel.

(Storage device 300)

The storage device 300 stores therein the images captured by the imaging element 21. The storage device 300 stores therein the image data that is generated by the imaging element 21 and that is on the images of the inside of the Petri dish 14 which are captured from before the cell group M is dispersed until at least a part of the cell group is suctioned. Further, the storage device 300 may store therein the positional data indicating the position identified by the position identification section 34. Moreover, the storage device 300 may store therein information for controlling each arm, such as the position of the extreme end of each arm before the arm control section 31 carries out an adjustment and the distance and speed of movement of the extreme end of each arm.

(Sample preparation method)

A sample preparation method is described. The sample preparation method is, for example, a sample preparation process in which the sample preparation system 100 is used. As illustrated in Fig. 3, in the sample preparation method, the following are carried out: (1) cell labeling; (2) enzymatic cell dispersion treatment (dispersing step); (3) cell dispersion (dispersing step); and (4) separation and collection of a single cell (collecting step). In the sample preparation method, from the process of (2) to the process of (4), the images of the inside of the Petri dish 14 in which the above processes are carried out are captured (image capturing step). Then, the position of each cell from (2) to (4) is identified with reference to the obtained images.

(1) Cell labeling

In order that capturing of the images of and tracking of the cell group M to be subjected to the processes are enabled, the cells are labeled. Examples of a method for labeling the cells include: a method in which the nuclei, cell membranes, cell organelles, nuclear membranes, cytoplasm, DNA, and the like of the cells are stained; a method in which light is emitted with use of a function of an enzyme in the cytoplasm; a method in which autofluorescence of the cells is used; and a method in which a fluorescent reporter gene is introduced.

In an example illustrated in Fig. 3, the cell group M in a culture medium is taken out, transferred to a container containing a stain so that the nuclei and the cell membranes are stained, and then transferred to a container containing a culture medium for washing so that the cell group M is washed. In this manner, the cells are labeled.

(2) Enzymatic cell dispersion treatment

In order that junctions between the cells in the cell group M are weaken, the cell group is treated with use of a cell dispersion enzyme. As the enzyme used in the enzymatic cell dispersion treatment, a conventionally known cell dispersion enzyme can be used, and examples thereof include trypsin, collagenase, dispase, papain, hyaluronidase, protease, pronase, and Accutase. Instead of the cell dispersion enzyme, a chelating agent, an antibody or a low molecular weight compound each of which, for example, inhibits a cell junction, or the like may be used. Examples of the chelating agent include EDTA which selectively binds with $Ca^{2+}$.

Note that in a case where the cell junctions in the original cell group M are weak, the (3) cell dispersion process may be carried out without carrying out the (2) enzymatic cell dispersion treatment. Note also that the order of the (2) enzymatic cell dispersion treatment and the (3) cell dispersion process may be switched such that the cell group M which has been subjected to the cell dispersion process is treated with use of the cell dispersion enzyme.

In the example illustrated in Fig. 3, the stained cell group M is transferred to the Petri dish 14 containing a solution that contains the dispersion enzyme, and left to stand for a given time so that the stained cell group M is subjected to the enzyme treatment. Images of the inside of the Petri dish 14 during the enzymatic cell dispersion treatment are captured by the imaging element 21, and image data on the cell group M during the enzyme treatment is obtained. Note that the imaging element 21 preferably captures the images of the cell group M from before the enzyme treatment.

(3) Cell dispersion

The cell group M treated with use of the cell dispersion enzyme is dispersed with use of the convex lens 12a of the dispersion arm 12. The convex lens 12a is moved to the top of the cell group M, moved in the direction of the bottom surface of the Petri dish 14, and pressed against the cell group M.

In the example illustrated in Fig. 3, the cell group M is a three-dimensional structure such as an organoid. Therefore, the convex lens 12a is pressed against the cell group M until the cell group M is dispersed on the two-dimensional plane. After the cell group M is dispersed on the two-dimensional plane, the convex lens 12a is shifted in the direction parallel to the bottom surface of the Petri dish 14, and moved to the direction away from the bottom surface. Images of the inside of the Petri dish 14 during the cell dispersion process are captured by the imaging element 21, and image data on the cell group M during the dispersion process is obtained.

(4) Separation and collection of single cell

A target single cell is separated from the dispersed cell group M, and the separated cell is collected. In the example illustrated in Fig. 3, the target single cell is disconnected and separated from the cell group M dispersed on the two-dimensional plane, by the needle 13a of the separation arm 13. Then, by suctioning the separated cell with use of the needle 11a of the suction arm 11, the target cell is collected. Images of the inside of the Petri dish 14 during the process of separating and collecting the single cell are captured by the imaging element 21, and image data on the cell group M during the process of separating and collecting the single cell is obtained.

In the sample preparation method, during the (2) enzymatic cell dispersion treatment, the (3) cell dispersion, and the (4) separation and collection of a single cell, out of the above steps of (1) to (4), the images of the inside of the Petri dish 14 in which the processes are carried out are obtained, and the cells in the Petri dish 14 are tracked. In the sample preparation method, it is possible to identify the position of each cell by generating, on the basis of the obtained images, a time-series image from when the cell group M is dispersed until the single cell is suctioned and then extracting and tracking the position of each cell at each time point. It is also possible to obtain information on the forms of the cells or changes in cells with time, by tracking the cells in the Petri dish 14 during the processes of (2) to (4).

(Examples of other cell dispersion processes)

In the sample preparation method, the cell dispersion process is not limited to a method in which the dispersion arm 12 is used, and another cell dispersion method may be used. As another cell dispersion method, a conventionally known cell dispersion method can be used, and examples thereof include: a method in the cells are vibrated; a method in which the cells are heated; a method in which the cells are left to stand in a dispersion enzyme solution; a method in which a flow of the solution is generated by, for example, shear stress; and a method in which an electric field or an supersonic wave is generated.

Examples of the method in which the cells are vibrated include a method in which an ultrasonic wave is used. Examples of the method in which the cells are heated include a method in which a material that easily absorbs heat (such as gold particles) is attached to the cell membranes and an electromagnetic wave is emitted. With regard to the method in which the cells are left to stand in the dispersion enzyme solution, in the cells subjected to dispersion enzyme treatment, an enzyme reaction progresses with elapse of time and dispersion of the cells proceeds. Therefore, by leaving the cells to stand until the cells are dispersed in a desired state, it is possible to carry out the cell dispersion process. As a result, it is possible to carry out not only a cell-by-cell analysis but also an analysis of, for example, a cell that functions systematically with an adjacent cell, an analysis of an interaction between adjacent cells, and the like.

[Analysis device 200]

The analysis device 200 is a device that analyzes the sample collected by the sample preparation system 100. As the analysis device 200, a conventionally known cell analysis device can be used. Examples of the sample analysis in the analysis device 200 include an RNA analysis, a genome analysis, a protein analysis, a metabolite analysis, and an analysis of a function such as a proliferation ability. Since the sample preparation system 100 can also collect a cell without destroying the cell, it is also possible to carry out a cell-by-cell analysis of a function in the analysis device 200. Further, since the sample preparation system 100 does not need to use a fluorescent protein to track a cell, it is also possible to carry out an analysis of a specimen of a human in the analysis device 200. An analysis in which the analysis device 200 is used can include continuously observing a cell or a cell mass as it is in a solution in which the cell group M is dispersed. Examples of such an analysis include an analysis of a division ability, a growth speed, motility, an infiltration ability, and the like of a cell or a cell mass, an ELISpot assay, and a comet assay. Moreover, the analysis device 200 carries out not only a cell-by-cell analysis but also an analysis of a sample that is considered to be a single functional unit in a cell mass, such as a pancreatic islet (cell mass-by-cell mass analysis).

In the example illustrated in Fig. 1, a cell is transported to the analysis device 200 in such a manner that the cell suctioned by the suction arm 11 of the sample preparation system 100 is discharged into each well of the plate 202 provided on the microscope stage 201. The analysis device 200 then subjects the transported cell to various sample analyses. Transport of a cell from the suction arm 11 to the plate 202 can be carried out while the cell is observed by a microscope having an objective lens 203.

In addition to obtaining a cell to be analyzed from the sample preparation system 100, the analysis device 200 can also obtain information on the original position, in the cell group M, of the target cell which original position has been identified in the sample preparation system 100, and use the information for a sample analysis. Therefore, it is possible to carry out not only a cell-by-cell analysis but also an analysis of, for example, a cell that functions systematically with an adjacent cell, an analysis of an interaction between adjacent cells, and the like.

(Software Implementation Example)

Control blocks of the control section 30 of the sample preparation system 100 may be realized by a logic circuit (hardware) provided in an integrated circuit (IC chip) or the like or may be alternatively realized by software.

In the latter case, the control section 30 of the sample preparation system 100 includes a computer that executes instructions of a program that is software realizing the foregoing functions. This computer includes, for example, at least one processor and a computer-readable recording medium that stores therein the program. The object of the present invention is be achieved by the processor of the computer reading the program from the recording medium and executing the program. Examples of the processor include a central processing unit (CPU). Examples of the recording medium include "non-transitory tangible media" such as a read only memory (ROM), a tape, a disk, a card, a semiconductor memory, and a programmable logic circuit. The computer may further include a random access memory (RAM) or the like in which the program is loaded. Further, the program may be made available to the computer via any transmission medium (such as a communication network and a broadcast wave) which allows the program to be transmitted. Note that an aspect of the present invention can also be realized in the form of a computer data signal in which the program is embodied via electronic transmission and which is embedded in a carrier wave.

Aspects of the present invention can also be expressed as follows:

A sample preparation system 100 includes: a microscope stage 22 on which a Petri dish (container) 14 in which a cell group M constituted by a plurality of cells that are joined to each other is held and in which the cell group M is dispersed is placed; a suction arm (collection arm) 11 which collects a sample from the dispersed cell group M; an imaging element 21 which captures microscope images of the inside of the Petri dish 14 from when the cell group M is dispersed until the sample is collected; and a position identification section 34 which identifies the position of the sample from before the cell group M is dispersed until the sample is collected, with reference to the microscope images.

According to the above configuration, the images of the inside of the Petri dish 14 are captured from when the cell group M is dispersed until the sample is collected from the cell group M. Therefore, it is possible to generate a time-series image from when the cell group M is dispersed until the sample is collected, on the basis of the images. Then, by extracting and tracking the position of the sample at each time point with reference to the generated time-series image, it is possible to identify the original position, in the cell group M, of the sample. Therefore, it is possible to collect the sample, and also possible to obtain positional information on the sample included in the cell group M.

Further, it is preferable that, in the sample preparation system 100, the cell group M be a three-dimensional structure and the suction arm 11 collect the sample from the cell group M dispersed on a two-dimensional plane in the Petri dish 14.

According to the above configuration, the cell group M, which is a three-dimensional structure, is dispersed on the two-dimensional plane in the Petri dish 14, and a cell is collected. Therefore, it is possible to easily collect a target cell. Moreover, images during dispersion of the cell group M, which is a three-dimensional structure, on the two-dimensional plane are also obtained. Therefore, it is possible to obtain three-dimensional positional information on the target cell in a three-dimensional structure of the cell group M.

Further, it is preferable that the sample preparation system 100 further include a dispersion arm 12 which has, at an extreme end thereof, a surface that presses the cell group M from the top of the cell group M in the direction of a bottom surface of the Petri dish 14.

According to the above configuration, by pressing a convex lens 12a at the extreme end of the dispersion arm 12 against the cell group M, it is possible to disperse the cell group M in the Petri dish 14. Furthermore, even in a case where the cell group M is a three-dimensional structure, it is possible to disperse the cell group M on the two-dimensional plane in the Petri dish 14, by pressing the convex lens 12a against the cell group M.

Further, it is preferable that, in the sample preparation system 100, the dispersion arm 12 have, at the extreme end thereof, a protruding part that protrudes in the direction in which the protruding part presses the cell group M.

According to the above configuration, the convex lens 12a at the extreme end of the dispersion arm 12 has the protruding part. Therefore, it is possible to appropriately press the cell group M, and also it is easy to carry out positioning of the convex lens 12a with respect to the cell group M.

It is preferable that the sample preparation system 100 further include an arm control section (adjustment mechanism) 31 which adjusts the position of the convex lens 12a at the extreme end of the dispersion arm 12 with respect to the cell group M.

According to the above configuration, the position of the convex lens 12a with respect to the cell group M is adjusted by the arm control section 31. Therefore, it is easy to adjust the position of the convex lens 12a with respect to the cell group M, and it is possible to appropriately press the convex lens 12a against the cell group M. This makes it possible to appropriately disperse the cell group M.

Further, it is preferable that, in the sample preparation system 100, the cell group M be held in the Petri dish 14 together with bead-like solid particles.

According to the above configuration, it is possible to observe a flow of a solution in the Petri dish 14 with reference to movement of the solid particles, and possible to appropriately carry out positioning of the convex lens 12a with reference to the flow of the solution.

Further, it is preferable that the sample preparation system 100 further include a storage device 300 which stores therein the images captured by the imaging element 21.

According to the above configuration, by generating the time-series image from image data stored in the storage device 300 and extracting and tracking the position of each cell at each time point, it is possible to use the images captured by the imaging element 21 to identify the position of each cell.

Further, it is preferable that the sample preparation system 100 further include a separation arm 13 which separates the sample from the dispersed cell group M and the suction arm 11 suction the sample separated by the separation arm 13.

According to the above configuration, even in a case where cell junctions of the cell group M dispersed in the Petri dish 14 are strong, it is easy to collect a target cell, because the cell disconnected by the separation arm 13 is suctioned by the suction arm 11.

A sample analysis system 1 includes any sample preparation system 100 described above and an analysis device 200 which analyzes a sample collected by the sample preparation system 100.

The sample analysis system 1 analyzes, in the analysis device 200, the sample collected in the sample preparation system 100. This makes it possible to carry out not only an analysis of a component obtained from a cell, such as an RNA analysis, but also a cell-by-cell analysis of a function with use of a cell collected without being destroyed. Furthermore, it is also possible to refer to, in a sample analysis in the analysis device 200, information on the original position, in a cell group M, of a target cell which original position has been identified in the sample preparation system 100.

A sample preparation method includes: a step of dispersing a cell group M constituted by a plurality of cells that are joined to each other; a step of collecting a sample from the dispersed cell group M; a step of capturing images from the step of dispersing the cell group M until the step of collecting the sample; and a step of identifying the position of the sample from the step of dispersing the cell group M until the step of collecting the sample, with reference to the captured images. This brings about an effect similar to that brought about by the above-described sample preparation system 100.

The present invention is not limited to the embodiments, but can be altered by a skilled person in the art within the scope of the claims. The present invention also encompasses, in its technical scope, any embodiment derived by combining technical means disclosed in differing embodiments.

[Supplementary notes]

The present invention can also be expressed as follows.

(Supplementary note 1)

In an aspect of the present invention, a sample preparation system including: a tracking section that associates a plurality of cells constituting a cell group with the respective plurality of cells constituting the cell group in which at least a part of junctions between adjacent ones of the plurality of cells is disconnected, in a dispersion process of (i) disconnecting at least the part of the junctions between the adjacent ones of the plurality of cells in the cell group in which the plurality of cells are joined to each other and (ii) dispersing the cell group.

(Supplementary note 2)

The sample preparation system described in Supplementary note 1, wherein the cell group is a three-dimensional structure.

(Supplementary note 3)

The sample preparation system described in Supplementary note 1 or 2, further including a dispersion arm that has an extreme end having a surface which is pressed against the cell group.

(Supplementary note 4)

The sample preparation system described in Supplementary note 3, wherein the dispersion arm has a protruding part on the surface which is pressed against the plurality of cells.

(Supplementary note 5)

The sample preparation system described in Supplementary note 3 or 4, further including an adjustment mechanism that adjusts a position of the extreme end of the dispersion arm with respect to the cell group.

(Supplementary note 6)

The sample preparation system described in any one of Supplementary notes 1 through 5, further including a microscope by which the cell group is observed in the dispersion process.

(Supplementary note 7)

The sample preparation system described in any one of Supplementary notes 1 through 6, further including a collection arm that collects a part of the cell group in which at least the part of the junctions between the adjacent ones of the plurality of cells is disconnected.

(Supplementary note 8)

The sample preparation system described in any one of Supplementary notes 1 through 7, further including a separation arm that separates a part of the cell group in which at least the part of the junctions between the adjacent ones of the plurality of cells is disconnected.

(Supplementary note 9)

A sample analysis system including: a sample preparation system described in any one of Supplementary notes 1 through 8; and an analysis device that analyzes a sample prepared by the sample preparation system.

(Supplementary note 10)

A sample preparation method including: a step of (i) disconnecting at least a part of junctions between adjacent ones of a plurality of cells in a cell group in which the plurality of cells are joined to each other and (ii) dispersing the cell group, the step being a step of dispersing the cell group while associating the plurality of cells constituting the cell group with the respective plurality of cells constituting the cell group in which at least the part of the junctions between the adjacent ones of the plurality of cells is disconnected.

Examples

[Example 1]

While positional information on each cell of a three-dimensional sample (organoid) was recorded, each cell was dispersed, tracked, and collected.

(1-1. Three arms used to operate sample)

Three sets of arms each of which could be controlled to move in the directions of three axes (x axis, y axis, and z axis) were disposed around a microscope stage (suction arm, dispersion arm, and separation arm). The following were attached to respective extreme ends of the arms: (i) a glass tube equipped with a pump for suction and discharge (inner diameter: 150 um, YODAKA Co., Ltd., A150S7522-6); (ii) a convex lens having a diameter of 2 mm and a radius of curvature of 1 mm (AS ONE Corporation, 3-6932-01); and (iii) a glass needle for cell separation (outer diameter: 10 $\mu$m, YODAKA Co., Ltd., A06S7522-6).

(1-2. Preparation of solutions)

Containers (MATSUNAMI GLASS IND., LTD., D11140H) containing respective solutions (sample liquid, stain, washing liquid, and cell dispersion liquid) shown in Table 1 were disposed on the microscope stage.

[Table 1]

| Sample liquid | Mouse-derived alveoli organoid + culture medium* |
|---|---|
| Stain | Culture medium containing nuclear staining reagent (5 $\mu$M, DRAQ5, BioStatus, Ltd.) and cell membrane staining reagent (1x, CellMask Green Plasma Membrane Stain, Thermo Fisher Scientific). |
| Washing liquid | Culture medium |
| Gell dispersion liquid | Solution obtained by adding 4.5 um polystyrene beads-(Polysciences, Inc.) to 0.6%, methylcellulose-containing cell dispersion enzyme solution (Accutase, Innovative Cell Technologies, Inc.) |
| *As the culture medium, used was a DMEM/F12 culture medium (NACALAI TESQUE, INC., 05177-15) containing 5% fetal bovine serum (Corning, 35-015-CV), sodium hydrogen carbonate (FUJIFILM Wako Pure Chemical Corporation, 195-16411), and antibiotics (penicillin-streptomycin (NACALAI TESQUE, INC., 26253-84)and amphotericin B (HyClone Laboratories, SV30078.01)). | |

(1-3. Determination of reference position of convex lens)

The microscope stage was moved so that the center of the container containing the cell dispersion liquid was moved to the center of a field of view. Then, the convex lens was immersed in the cell dispersion liquid. In a case where a convex lens is moved up or down in a solution, polystyrene beads in the solution move in a centripetal or centrifugal direction around the lowest point of the lens on an xy plane near a bottom surface of a container. Thus, the lowest point of the convex lens was identified by observing such movement of beads (flow of the solution), and the position of the convex lens was adjusted in the x axis direction and the y axis direction so that the lowest point of the convex lens was located at the center of a field of microscope. Thereafter, a Newton's rings-like image observed when the convex lens was brought into contact with (brought close to) the bottom of the container was used as an index to finely adjust the position of the convex lens in the x axis direction, the y axis direction, and the z axis direction.

Specifically, the height of the convex lens was gradually lowered in the solution. Then, a position at which a Newton's rings-like image was clearly observed for the first time (position at which the lowest point of the convex lens was brought into contact with the bottom of the container and vibration or the like of the convex lens subsided) was determined. Moreover, the center of the observed image was finely adjusted so that the center of the image was located at the center of the field of microscope, and the position of the convex lens in the x axis direction and the y axis direction was determined. The position of the convex lens in the x axis direction, the y axis direction, and the z axis direction at that time was used as a reference position of the convex lens. After the reference position was determined, the convex lens was moved outside the container, and held at a waiting position at which operations of suctioning and discharging the sample and movement of the microscope stage were not hindered.

(1-4. Staining of sample)

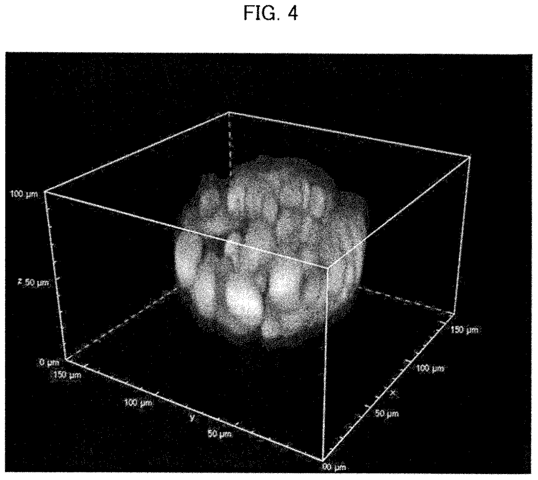

While the microscope stage was moved and the inside of the sample liquid was observed, the target sample was suctioned with use of the glass tube, discharged into the container containing the stain, and then stained at room temperature for 15 minutes. After the sample was stained, the sample was suctioned again with use of the glass tube. The sample was discharged into the container containing the washing liquid, and then an excess stain was removed. Thereafter, a confocal laser microscope system (inverted microscope ECLIPSE Ti2-E, confocal unit A1R HD25, Nikon) was used to obtain a three-dimensional fluorescence image of the stained sample. Fig. 4 shows the obtained three-dimensional fluorescence image (intervals in the z axis: 5 $\mu$m, number of slices: 20 slices) of cell nuclei immediately after the staining.

(1-5. Enzymatic cell dispersion treatment)

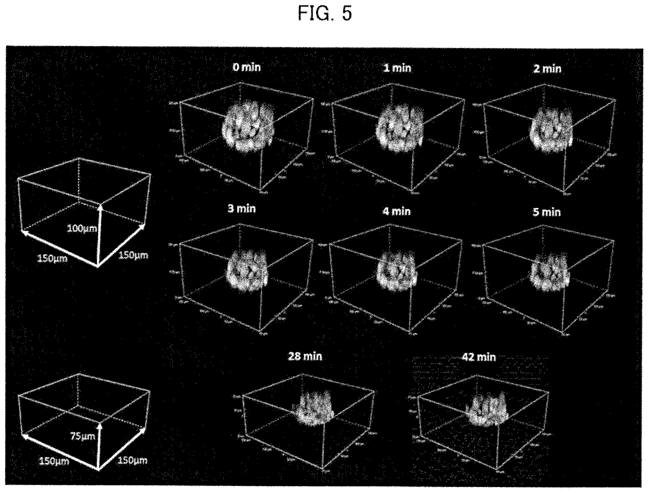

The stained sample was suctioned with use of the glass tube and discharged into the container containing the cell dispersion liquid. Thereafter, the microscope stage was moved so that the sample was located at the center of the field of view. At the same time, the convex lens was moved from the waiting position, and was left to stand still at a point 200 $\mu$m above the reference position determined in the above 1-3. Immediately after movement of the convex lens was completed, time-lapse capturing of three-dimensional fluorescence images was started. For the first 5 minutes, images were obtained every other minute. Thereafter, images were further obtained after 28 minutes and 42 minutes. Fig. 5 shows obtained changes in position of the cell nuclei with elapse of an enzymatic cell dispersion treatment time.

(1-6. Cell dispersion by convex lens)

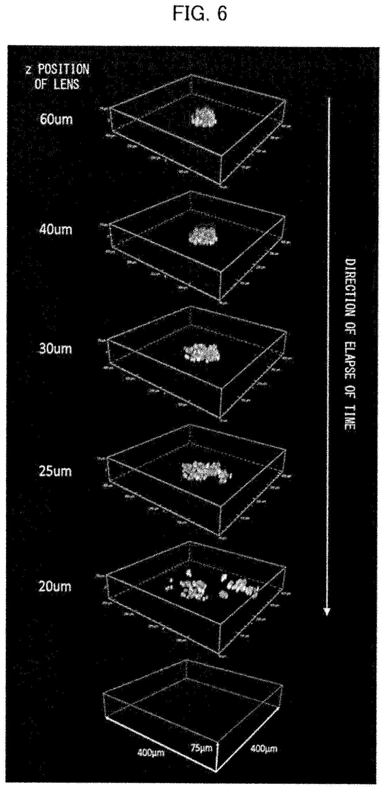

After the enzymatic cell dispersion treatment, the convex lens was gradually lowered while the sample was observed in a bright field. Then, a three-dimensional fluorescence image was obtained at a position at which the convex lens approached the sample and the shape of the sample began to change (height: 60 pm). Thereafter, with use of a program function of a motorized manipulator, the position of the lens was gradually lowered by repeating the following operation: "the lens was moved 5 $\mu$m below the original position by repeating, a total of 5 times, an up and down movement of moving the lens +2 $\mu$m and moving the lens -3 um in the z direction at a speed of 10 $\mu$m / sec". In so doing, three-dimensional fluorescence images were respectively obtained at heights of 50 $\mu$m, 40 $\mu$m, 30 $\mu$m, 25 $\mu$m, and 20 $\mu$m. A manner in which each cell constituting the three-dimensional sample (organoid) was dispersed and disposed on the two-dimensional plane by the movement of the convex lens was thus recorded. Fig. 6 shows changes in position of the cells in the process of dispersing the cells with use of the convex lens.

(1-7. Movement and removal of convex lens)

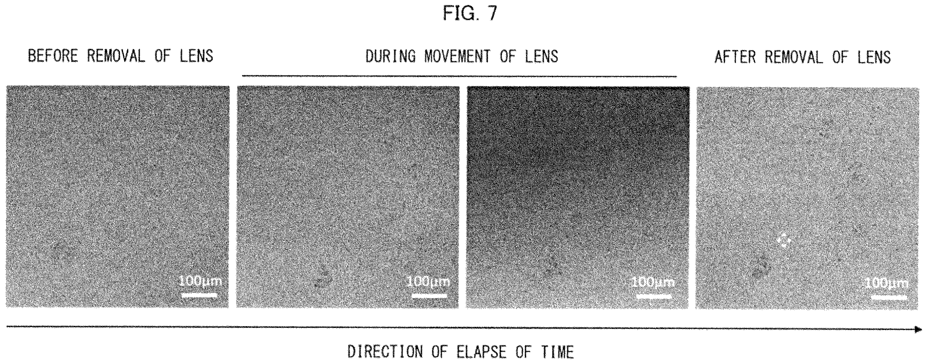

The convex lens located on the dispersed sample was moved out of the solution. While the convex lens was moved, bright field observation images were captured as a moving image with use of a CMOS camera (Zyla, Andor). In this manner, movement of the cells with the movement of the convex lens was recorded. In so doing, a direction and a speed of the movement of the convex lens were controlled to fall within a range in which the movement of the cells (distance, speed, and the like) could be tracked with the moving image. Specifically, the lens was moved 100 um upward at 0.4 $\mu$m/sec in the z axis direction, translated approximately 2 mm at 20 $\mu$m/sec in the y axis direction, moved upward again at 20 $\mu$m/sec in the z axis direction, and then pulled out of a liquid surface. Fig. 7 shows changes in position of the cells with the movement of the lens.

(1-8. Separation and collection of single cell)

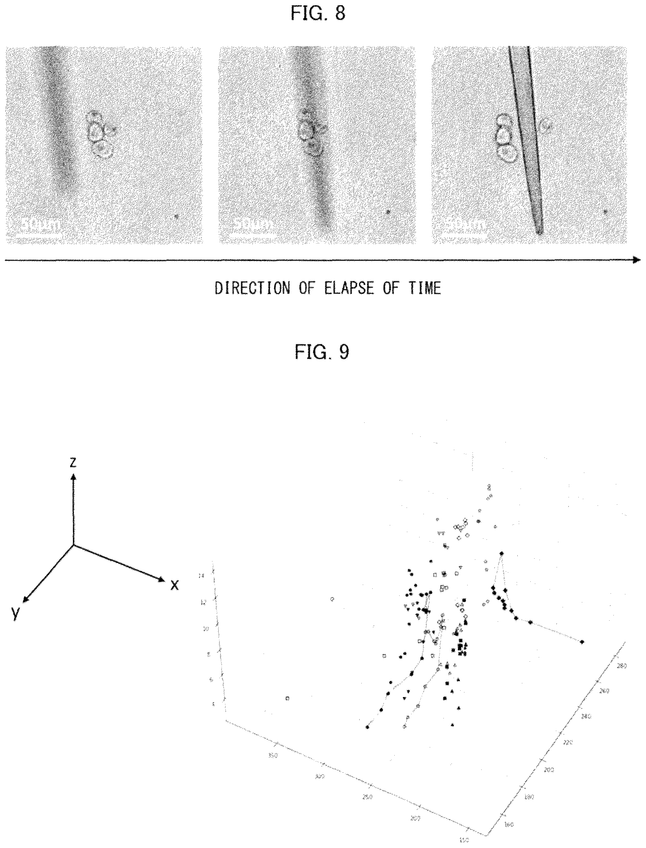

The glass needle for cell separation was operated so as to disconnect cell junctions and separate each cell in a cell mass of the sample dispersed on the two-dimensional plane (Fig. 8). A separated cell was suctioned with use of the glass tube, and then discharged and collected in a PCR tube. A manner in which the cell was separated and collected was recorded by capturing bright field observation images as a moving image with use of a CMOS-camera (Zyla, Andor). Fig. 8 is shows a manner in which the single cell was separated from the cell mass of the sample with use of the glass needle for cell separation.

(1-9. Tracking of cells)

The time-series images (moving image) obtained in 1-5. to 1-8. were analyzed. With use of image integration software NIS-Elements C (Nikon), images processing (noise reduction by Denoise.AI) was carried out. Thereafter, a three-dimensionally reconstructed time-series image was generated. While the generated time-series image was reversely reproduced, the position of the collected cell at each time point was extracted and tracked, and the three-dimensional position, in the sample before dispersion, of the collected cell was identified. Fig. 9 shows the three-dimensional position and the track of movement of the separated and collected cell in the dispersion process. In Fig. 9, pixel coordinates (x, y) of the cell nucleus (the vicinity of the center) of each cell and slice numbers (z) in the z axis direction at a time of capturing of the images, the pixel coordinates and the slice numbers being recorded in 1-5. and 1-6., are three-dimensionally plotted. Markers in Fig. 9 indicate movement points of cells (12 cells) that are considered identical. As an example, tracks of movement of three cells are shown with use of solid lines.

As illustrated in Fig. 9, it was shown that, with regard to each cell in a cell population that was the sample, it was possible to obtain positional information at each time point during cell dispersion, separation, and collection and possible to track a track of movement of each cell. It was also possible to suitably identify and track the three-dimensional position of each cell in a three-dimensional sample.

[Example 2]

While positional information on each cell of a three-dimensional sample (organoid) was recorded, each cell was dispersed, and disposed on a two-dimensional plane. In the present example, a suction arm and a dispersion arm were used to carry out processes up to cell dispersion, as described below.

(2-1. Two arms used to operate sample)

Two sets of arms each of which could be controlled to move in the directions of three axes (x axis, y axis, and z axis) were disposed around a microscope stage (suction arm and dispersion arm). The following were attached to respective extreme ends of the arms: (i) a glass tube equipped with a pump for suction and discharge (inner diameter: 150 um, YODAKA Co., Ltd., A150S7522-6); and (ii) a convex lens having a diameter of 2 mm and a radius of curvature of 1 mm (AS ONE Corporation, 3-6932-01).

(2-2. Preparation of containers)

In the present example, a container for staining of the sample and a container for dispersion of the sample (MATSUNAMI GLASS IND., LTD., D141400) which were each coated with poly-L-lysine (Sigma-Aldrich, P8920) were used. Together with a container for the sample (MATSUNAMI GLASS IND., LTD., D11140H), three containers were disposed on the microscope stage.

(2-3. Determination of reference position of convex lens)

The microscope stage was moved so that a central part of a well of the container for dispersion was moved to the center of a field of view. In the present example. By moving the dispersion arm, the position of the convex lens in the x axis direction and the y axis direction was adjusted so that the lowest point of the convex lens was located at the center of a field of microscope. Thereafter, a Newton's rings-like image observed when the convex lens was brought into contact with (brought close to) the bottom of the container was used as an index to finely adjust the position of the convex lens in the x axis direction, the y axis direction, and the z axis direction. Specifically, positioning of the convex lens was carried out in a manner similar to that in Example 1, except that the positioning of the convex lens was carried out without putting a dispersion liquid in the container for dispersion, and a reference position was determined.

(2-4. Preparation of solutions)

Solutions (sample liquid, stain, washing liquid, cell dispersion liquid A, cell dispersion liquid B, and diluent) shown in Table 2 below were prepared. The sample liquid was put in the container for the sample. The stain and the washing liquid were put in respective different wells of the container for staining. The cell dispersion liquid A was put in the container for dispersion.

[Table 2]

| Sample liquids | Mouse-derived alveoli organoid + culture medium* |
|---|---|
| Stain | Culture medium containing nuclear staining reagent (5- uM, DRAQ5, BioStatus, Ltd.) and cell membrane staining reagent (1x, CellMask, Green Plasma Membrane Stain, Thermo Fisher Scientific) |
| Washing liquid | Culture medium |
| Cell dispersion liquid A | Dulbecco's phosphate buffered saline (NACALAI TESQUE, INC., 14249-24) containing 0. 1 mM EDTA (gibco, 15575-038) |
| Cell dispersion liquid B | Hanks' balanced salt solution (NACALAI TESQUE, INC., 09735-75) containing pronase (Roche, 10165921001) |
| Diluent | Culture medium |
| *As the culture medium, used was a DMEM/F12 culture medium (NACALAI TESQUE, INC., 05177-15) containing 5% fetal bovine serum (Corning, 35-015-CV), sodium hydrogen carbonate (FUJIFILM Wako Pure Chemical Corporation, 195-16411), and antibiotics (penicillin-streptomycin (NACALAI TESQUE, INC., 26253-84), and amphotericin B (HyClone Laboratories, SV30078.01)). | |

(2-5. Staining of sample)

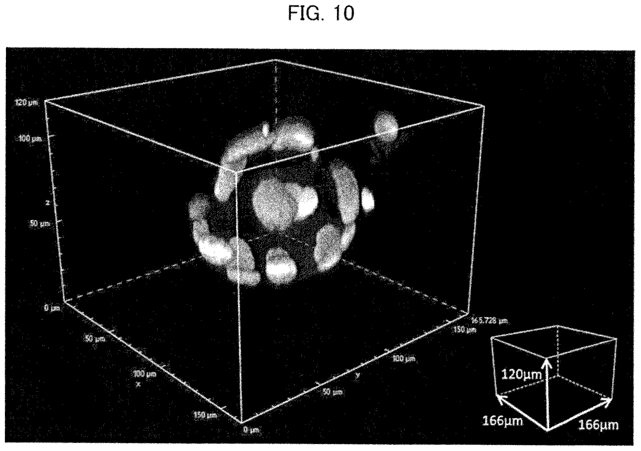

While the microscope stage was moved and the inside of the sample liquid was observed, the target sample was suctioned with use of the glass tube, discharged into the well which was of the container for staining and which contained the stain, and then stained at room temperature for 20 minutes. After the sample was stained, the sample was suctioned again with use of the glass tube. The sample was discharged into the well containing the washing liquid, and an excess stain was removed. Thereafter, a confocal laser microscope system (inverted microscope ECLIPSE Ti2-E, confocal unit AIR HD25, Nikon) was used to obtain a three-dimensional fluorescence image of the stained sample. Fig. 10 shows the obtained three-dimensional fluorescence image (intervals in the z axis: 2 $\mu$m, number of slices: 60 slices, a noise reduction process had been carried out by Denoise.ai) of cell nuclei immediately after the staining.

(2-6. Cell dispersion solution treatment)

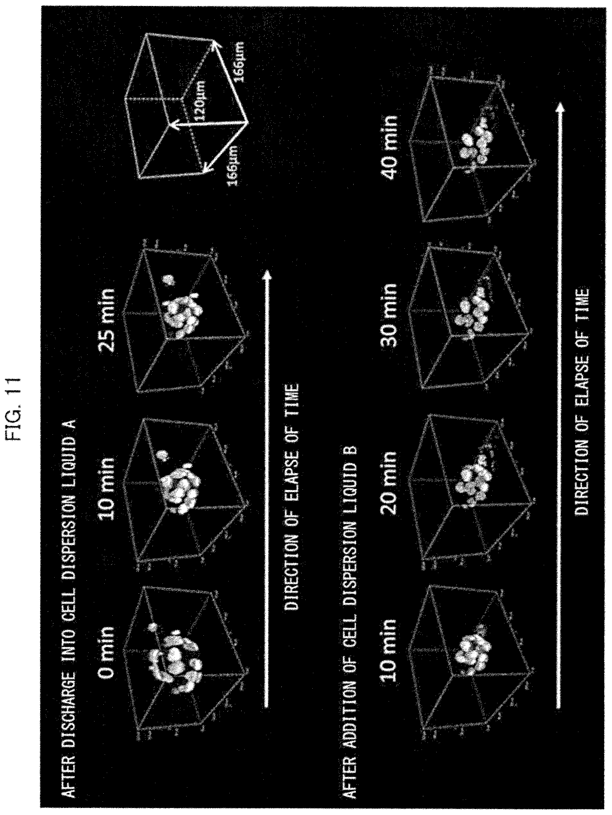

In the present example, images while the cells were treated with use of the dispersion liquids were obtained, while the cell dispersion liquid B and the diluent were added to the container for dispersion. Specifically, the stained sample was suctioned with use of the glass tube and discharged into the container which was for dispersion and which contained the cell dispersion liquid A. Thereafter, the microscope stage was moved so that the sample was located at the center of the field of view. Then, three-dimensional fluorescence images were obtained after 0 minutes, 10 minutes, and 25 minutes. Immediately after an image was obtained after 25 minutes, the cell dispersion liquid B was gently poured with use of a micropipette such that the sample did not move. After the cell dispersion liquid B was added, three-dimensional fluorescence images were obtained when 10 minutes, 20 minutes, 30 minutes, and 40 minutes elapsed. After capturing of the images were completed, the diluent was gently poured with use of a micropipette, and the sample was left to stand for 30 minutes. Fig. 11 shows obtained changes in position of the cell nuclei with elapse of a cell dispersion solution treatment time.

(2-7. Cell dispersion by convex lens)

In the present example, after the cells were disposed on the two-dimensional plane, the cells were further dispersed on the two-dimensional plane with use of the convex lens.
Specifically, after the diluent was added and the sample was left to stand, the convex lens was moved from a waiting position and moved to a point 200 um above the reference position determined in 2-3. The convex lens was gradually lowered while the sample was observed in a bright field. Then, a three-dimensional fluorescence image was obtained at a position at which the convex lens approached the sample and began to be in contact with the top of the sample (height: 75 pm). Thereafter, with use of a program function of a motorized manipulator, the position of the lens was gradually lowered by repeating the following operation: "the lens was moved 5 $\mu$m below the original position by repeating, a total of 5 times, an up and down movement of moving the lens +2 $\mu$m and moving the lens -3 um in the z axis direction at a speed of 10 $\mu$m/sec". In so doing, three-dimensional fluorescence images were respectively obtained at heights of 60 $\mu$m, 50 $\mu$m, 40 $\mu$m, and 35 $\mu$m.

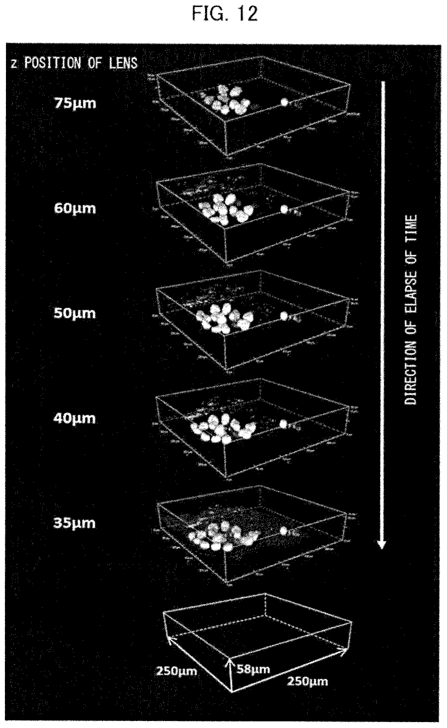

A manner in which each cell constituting the three-dimensional sample (organoid) was dispersed and disposed on the two-dimensional plane by the movement of the convex lens was thus recorded. By this recording, individual cells moving from positions in a three-dimensional space to positions on the two-dimensional plane were tracked. Fig. 12 shows changes in position of the cells in the process of dispersing the cells with use of the convex lens.

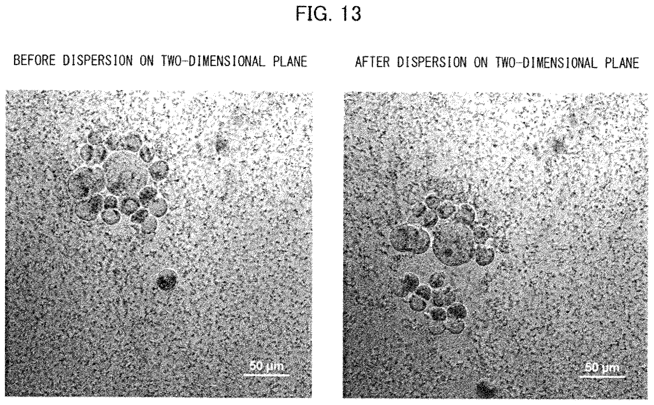

Furthermore, while the positions of the cells which had been disposed on the two-dimensional plane were recorded in bright field images, the following operation was carried out with use of a program function of a motorized manipulator: "an up and down movement was repeated in a range of 2 $\mu$m in the z axis direction at a speed of 8 $\mu$m/sec". Simultaneously with this operation, the microscope stage was moved so that the cells were further dispersed on the two-dimensional plane. Fig. 13 shows changes in position of the cells before and after dispersion on the two-dimensional plane.

Industrial Applicability

The present invention can be used in a pharmaceutical field and the like by being used in a cell analysis.

Reference Signs List

1       Sample analysis system
M       Cell group
10      Sample preparation robot
11      Suction arm (collection arm)
12      Dispersion arm
12a     Convex lens
13      Separation arm
14      Petri dish (container)
21      Imaging element
22      Microscope stage
31      Arm control section (adjustment mechanism)
34      Position identification section (tracking section)
100     Sample preparation system
200     Analysis device
300     Storage device

**Claims**

1. A sample preparation system comprising:
   a tracking section that associates a plurality of cells constituting a cell group with the respective plurality of cells constituting the cell group in which at least a part of junctions between adjacent ones of the plurality of cells is disconnected, in a dispersion process of (i) disconnecting at least the part of the junctions between the adjacent ones of the plurality of cells in the cell group in which the plurality of cells are joined to each other and (ii) dispersing the cell group.

2. The sample preparation system as set forth in claim 1, wherein the cell group is a three-dimensional structure.

3. The sample preparation system as set forth in claim 1 or 2, further comprising a dispersion arm that has an extreme end having a surface which is pressed against the cell group.

4. The sample preparation system as set forth in claim 3, wherein the dispersion arm has a protruding part on the surface which is pressed against the plurality of cells.

5. The sample preparation system as set forth in claim 3, further comprising an adjustment mechanism that adjusts a position of the extreme end of the dispersion arm with respect to the cell group.

6. The sample preparation system as set forth in claim 1 or 2, further comprising a microscope by which the cell group is observed in the dispersion process.

7. The sample preparation system as set forth in claim 1 or 2, further comprising a collection arm that collects a part of the cell group in which at least the part of the junctions between the adjacent ones of the plurality of cells is disconnected.

8. The sample preparation system as set forth in claim 1 or 2, further comprising a separation arm that separates a part of the cell group in which at least the part of the junctions between the adjacent ones of the plurality of cells is disconnected.

9. A sample analysis system comprising:

   a sample preparation system recited in claim 1 or 2; and
   an analysis device that analyzes a sample prepared by the sample preparation system.

10. A sample preparation method comprising:
   a step of (i) disconnecting at least a part of junctions between adjacent ones of a plurality of cells in a cell group in which the plurality of cells are joined to each other and (ii) dispersing the cell group, the step being a step of dispersing the cell group while associating the plurality of cells constituting the cell group with the respective plurality of cells constituting the cell group in which at least the part of the junctions between the adjacent ones of the plurality of cells is disconnected.

EP 4 442 809 A1

FIG. 1

FIG. 2

FIG. 3

(1) CELL LABELING

(2) CELL DISPERSION ENZYME TREATMENT

CULTURE MEDIUM ~M

STAIN ~M

CULTURE MEDIUM (FOR WASHING) ~M

DISPERSION ENZYME ~M

(3) CELL DISPERSION

12
12a
14
M

12
12a
14
M

12
12a
14
M

(4) SEPARATION AND COLLECTION OF SINGLE CELL

13a
M

11a
M

EP 4 442 809 A1

FIG. 4

FIG. 5

FIG. 6

FIG. 7

BEFORE REMOVAL OF LENS          DURING MOVEMENT OF LENS          AFTER REMOVAL OF LENS

DIRECTION OF ELAPSE OF TIME

EP 4 442 809 A1

FIG. 8

DIRECTION OF ELAPSE OF TIME

FIG. 9

FIG. 10

FIG. 11

EP 4 442 809 A1

FIG. 12

## FIG. 13

BEFORE DISPERSION ON TWO-DIMENSIONAL PLANE    AFTER DISPERSION ON TWO-DIMENSIONAL PLANE

## INTERNATIONAL SEARCH REPORT

International application No.

**PCT/JP2022/043479**

**A.    CLASSIFICATION OF SUBJECT MATTER**

*C12N 5/07*(2010.01)i; *G01N 1/28*(2006.01)i; *C12M 1/00*(2006.01)i
FI:    C12M1/00 A; C12N5/07; G01N1/28 F; G01N1/28 J; G01N1/28 G

According to International Patent Classification (IPC) or to both national classification and IPC

**B.    FIELDS SEARCHED**

Minimum documentation searched (classification system followed by classification symbols)

C12N5/07; G01N1/28; C12M1/00

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

**C.    DOCUMENTS CONSIDERED TO BE RELEVANT**

| Category* | Citation of document, with indication, where appropriate, of the relevant passages | Relevant to claim No. |
|---|---|---|
| X | JP 2003-339373 A (NATIONAL INSTITUTE OF ADVANCED INDUSTRIAL SCIENCE AND TECHNOLOGY) 02 December 2003 (2003-12-02) <br> claim 9, paragraphs [0010], [0013]-[0016] | 1, 2, 6 |
| Y | claim 9, paragraphs [0010], [0013]-[0016] | 1-10 |
| X | JP 2013-202016 A (KANAGAWA ACADEMY OF SCIENCE & TECHNOLOGY) 07 October 2013 (2013-10-07) <br> paragraphs [0037]-[0040], fig. 5, 6 | 1, 6, 7 |
| Y | paragraphs [0037]-[0040], fig. 5, 6 | 1-10 |
| X | WO 2011/161962 A1 (KAWASAKI JUKOGYO KABUSHIKI KAISHA) 29 December 2011 (2011-12-29) <br> claims 7, 17, paragraphs [0048]-[0049], fig. 1 | 1, 2 |
| Y | claims 7, 17, paragraphs [0048]-[0049], fig. 1 | 1-10 |

☑ Further documents are listed in the continuation of Box C.       ☑ See patent family annex.

\*    Special categories of cited documents:
"A"    document defining the general state of the art which is not considered to be of particular relevance
"E"    earlier application or patent but published on or after the international filing date
"L"    document which may throw doubts on priority claim(s) or which is cited to establish the publication date of another citation or other special reason (as specified)
"O"    document referring to an oral disclosure, use, exhibition or other means
"P"    document published prior to the international filing date but later than the priority date claimed

"T"    later document published after the international filing date or priority date and not in conflict with the application but cited to understand the principle or theory underlying the invention
"X"    document of particular relevance; the claimed invention cannot be considered novel or cannot be considered to involve an inventive step when the document is taken alone
"Y"    document of particular relevance; the claimed invention cannot be considered to involve an inventive step when the document is combined with one or more other such documents, such combination being obvious to a person skilled in the art
"&"    document member of the same patent family

| Date of the actual completion of the international search | Date of mailing of the international search report |
|---|---|
| **02 February 2023** | **14 February 2023** |

| Name and mailing address of the ISA/JP | Authorized officer |
|---|---|
| **Japan Patent Office (ISA/JP)** <br> **3-4-3 Kasumigaseki, Chiyoda-ku, Tokyo 100-8915** <br> **Japan** | |
| | Telephone No. |

Form PCT/ISA/210 (second sheet) (January 2015)

## INTERNATIONAL SEARCH REPORT

| | International application No. |
|---|---|
| | **PCT/JP2022/043479** |

### C. DOCUMENTS CONSIDERED TO BE RELEVANT

| Category* | Citation of document, with indication, where appropriate, of the relevant passages | Relevant to claim No. |
|---|---|---|
| X | WO 2020/153837 A1 (ERASMUS UNIVERSITY MEDICAL CENTER ROTTERDAM) 30 July 2020 (2020-07-30) | 1, 2, 6, 7 |
| | p. 2, lines 1-15, p. 10, line 20 to p. 11, line 3, p. 12, lines 4-15, fig. 3C | |
| Y | p. 2, lines 1-15, p. 10, line 20 to p. 11, line 3, p. 12, lines 4-15, fig. 3C | 1-10 |
| A | WO 2016/013392 A1 (HITACHI HIGH TECH CORP) 28 January 2016 (2016-01-28) paragraphs [0006], [0017] | 1-10 |
| A | JP 2017-124452 A (NSK LTD) 20 July 2017 (2017-07-20) claims 1-2, 4, paragraph [0044], fig. 1 | 1-10 |

Form PCT/ISA/210 (second sheet) (January 2015)

33

## INTERNATIONAL SEARCH REPORT
**Information on patent family members**

International application No.

**PCT/JP2022/043479**

| Patent document cited in search report | | | Publication date (day/month/year) | Patent family member(s) | | | Publication date (day/month/year) |
|---|---|---|---|---|---|---|---|
| JP | 2003-339373 | A | 02 December 2003 | US | 2003/0219889 | A1 | |
| | | | | claim 9, paragraphs [0061], [0068]-[0079] | | | |
| | | | | EP | 1365017 | A1 | |
| JP | 2013-202016 | A | 07 October 2013 | WO | 2013/146971 | A1 | |
| | | | | paragraphs [0037]-[0040], fig. 5, 6 | | | |
| WO | 2011/161962 | A1 | 29 December 2011 | US | 2012/0315620 | A1 | |
| | | | | claims 7, 17, paragraphs [0061]-[0062], fig. 4 | | | |
| | | | | EP | 2487249 | A1 | |
| | | | | JP | 2016-28607 | A | |
| WO | 2020/153837 | A1 | 30 July 2020 | US | 2022/0090007 | A1 | |
| | | | | paragraphs [0005], [0036], [0040], fig. 3C | | | |
| | | | | EP | 3686273 | A1 | |
| WO | 2016/013392 | A1 | 28 January 2016 | US | 2018/0080002 | A1 | |
| | | | | paragraphs [0007], [0082] | | | |
| JP | 2017-124452 | A | 20 July 2017 | (Family: none) | | | |

Form PCT/ISA/210 (patent family annex) (January 2015)

34

**EP 4 442 809 A1**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2018068169 A